# EUROPEAN PATENT SPECIFICATION

(11) **EP 0 389 102 B1**
(45) Date of publication and mention of the grant of the patent: **10.05.1995**
(21) Application number: 90301772.1
(22) Date of filing: 19.02.1990
(51) Int. Cl.: A61B 17/08

(54) **Skin fastener**
Hautklammer
Agrafe pour la peau

(30) Priority: 22.02.1989 US 314368; 27.06.1989 US 372025
(43) Date of publication of application: 26.09.1990
(60) Divisional application: 92122148.7
(73) Proprietor: United States Surgical Corporation, Norwalk, Connecticut 06856 (US)
(72) Inventor: Green, David T., Westport, Connecticut 06880 (US)
(74) Representative: Marsh, Roy David

(56) References cited:
- US-A- 3 209 754
- US-A- 3 273 562
- US-A- 4 217 902
- US-A- 4 635 637

## Description

This invention relates to fasteners, and particularly to surgical fasteners used to join body tissue.

Fasteners have been used surgically to eliminate the need for suturing, which is both time consuming and inconvenient. In many applications the surgeon can use a stapler apparatus, i.e., a fastener implanting device loaded with surgical fasteners to accomplish in a few seconds what would have taken many minutes to perform by suturing. This reduces blood loss and trauma to the patient. The terms "fastener" and "staple" are used interchangeably herein.

Surgical fasteners have been in the form of ordinary metal staples, which are bent by the delivery apparatus to hook together body tissue. Also, two-part fasteners have been used, as illustrated in U.S. Patent No. 4,506,670 in which a barbed staple is used in conjunction with a retaining piece to hold the staple in place.

Typically, the two part staple comprises a back span and two barbed prongs which are engaged and locked into a separate retainer piece. In use, the staple is pressed into the body tissue so that the barbs penetrate the tissue and emerge from the other side where they are then locked into the retainer piece. The retainers prevent the staple from working loose from the tissue. The two piece fasteners cannot be unlocked and are not removable. For this reason, they must be made of a bioabsorbable material. Korthoff et al, US Patent No.4,667,674 discloses a two part surgical fastener comprising a fastener member and a retainer member. The fastener member has a base, and a pair of prongs extending perpendicularly from the base. The prongs are spaced inward from the respective ends of the base in order to prevent splaying of the prongs, and to improve hemostasis.

The two piece fasteners require the staple delivery apparatus to have access to both sides of the tissue. Usually, such devices have a U-shaped member into which tissue is inserted. The stapler apparatus has a fastener holder and an anvil which are pivotally connected at one end, and mounted on the legs of the U-shaped support structure. See, for example, Green U.S. Patent No. 4,402,445, which discloses a surgical fastener and means for applying same. In a surgical operation, the tissue to be joined is positioned between the fastener holder and the anvil, which contains the fastener retainers. The fasteners are ejected from the holder into the tissue, and the prongs are locked into the retainers.

Certain types of wounds or incisions in fascia tissue require that the two edges of the wound or incision be held together in close approximation in order to promote proper healing. The fasteners described in the above-cited patents are designed for preventing bleeding by improving hemostatis, but they are not designed for holding two edges of a wound together. Green, U.S. Patent No. 4,610,250 discloses a two part surgical fastener in which the retainer portion has a camming surface for bending two prongs of the fastener inward. When the fastener is properly positioned across a wound the bent prongs will maintain the edges of the wound in close adjacency until the fastener is biologically absorbed. This fastener, too, requires an applying instrument to have access to both sides of the body tissue.

In some applications, however, it is not possible to have access to body tissue from two opposite directions. For example, in skin grafting applications one can only apply fasteners from a stapler positioned above the skin.

The prior art includes many examples of surgical staplers which do not enclose the body tissue between an anvil and fastener holder. For example, surgical staplers such as those described in U.S. Patent No. 3,643,851 and U.S. Patent No. 4,618,086 approach the skin from one direction. However, they require the use of staples which are malleable enough to be crimped by an anvil so that the prongs hook into the tissue. Typically, such staples are made of metal and are not bioabsorbable. They must be removed by another device, such as a stapler extractor. Furthermore, such staples are useful for joining tissue layers laterally, as for example in closing wounds in skin or fascia, and, although useable, they are not optimum for laminarly fastening one layer of tissue onto another as in skin grafting.

US-A-4635637 discloses a surgical fastener for joining layers of body tissue. The fastener has a backspan and two prongs extending substantially perpendicular to the backspan and parallel to each other, each prong having a shaft with a triangular barb to retain the fastener in body tissue without a separate retainer.

EP-A-0138687 discloses such a surgical fastener in combination with a retainer. The fastener is bioabsorbable and the prongs are spaced apart from the ends of the backspan.

Hence there is a need for a bioabsorbable surgical fastener which is capable of being delivered into the surface of body tissue from one direction, which can join layers of tissue both laterally and laminarly as required in skin grafting applications, which provides a biasing force to hold adjacent edges of body tissue in close approximation, and which does not have to be removed from the body (a painful procedure).

### SUMMARY OF THE INVENTION

Provided herein is a surgical fastener for joining layers of body tissue. The fastener comprises: a back span, and at least two prongs extending substantially perpendicularly from said backspan in substantially parallel relationship to each other, each prong having a shaft portion and at least one flat, substantially triangular shaped barb means for penetrating body tissue, and retaining the fastener in the body tissue.

In accordance with the invention, the fastener is bioabsorbable and is retained in body tissue without the need for a separate retainer. The prongs are spaced apart from the ends of the backspan and the barbs are blade-like and thin relative to the respective prong.

In an alternative embodiment the surgical fastener comprises: a backspan comprising a bridge member; and at least two prongs depending from the backspan, said prongs being in oblique orientation to each other in which the distal ends of said prongs are inclined towards each other, said prongs being movable in response to pressure to a substantially parallel orientation to each other, and being resiliently returnable to said oblique orientation when said pressure is removed.

The surgical fasteners of the present invention are optimally constructed of a bioabsorbable resinous material. A preferred resinous material, which is absorbable in the body, is disclosed in Kaplan et al., U.S. Patent No. 4,523,591.

Further features of the invention, its nature and various advantages will be more apparent from the accompanying drawings and the following detailed description of the invention.

In the accompanying drawings:
Figs. 1a, 1b and 1c illustrate in side, bottom and end views respectively, an embodiment of the present invention;
Figs. 2a, 2b and 2c illustrate in side, bottom and end views respectively an alternative embodiment of the invention having side indentations;
Figs. 3a, 3b and 3c illustrate in side, bottom and end views respectively an alternative embodiment of the present invention having a barb on only one side of each prong;
Figs. 4a, 4b and 4c illustrate in side, bottom and end views respectively, an alternative embodiment of the invention having multiple rows of barbs on each prong;
Figs. 5a and 5b illustrate a view of the fastener of the present invention within the ejection chamber of a fastener implanting instrument;
Fig. 6 illustrates a side view showing the fastener of the present invention in conjunction with a pusher member of a fastener implanting instrument;
Fig. 7 illustrates a sectional side view showing the implanted fastener in use; and
Fig. 8 shows a detailed view of the prong of the present fastener invention.
Fig. 9a illustrates a front view of the inwardly biased skin fastener;
Fig. 9b illustrates a top view of the inwardly biased skin fastener;
Fig. 9c illustrates a bottom view of the inwardly biased skin fastener;
Fig. 9d illustrates a detailed bottom view of the prong and barb;
Fig. 10a illustrates a front view of an alternative embodiment of the present invention;
Fig. 10b illustrates a top view of the alternative embodiment:
Fig. 10c illustrates a bottom view of the alternative embodiment;
Fig. 11 illustrates a diagrammatic view of a skin fastener of the present invention in conjunction with the firing chamber of a fastener implanting instrument;
Figs. 12 and 13 illustrate perspective views of alternative embodiments of the inwardly biased skin fastener which do not possess arm members.

The skin fastener or skin tack as illustrated can be used in conjunction with a surgical stapling instrument capable of propelling or implanting the skin fastener into the body tissue to be fastened. Generally such instruments will have a chamber for holding one or more fasteners, a means for storing and releasing power such as a spring, and a trigger means.

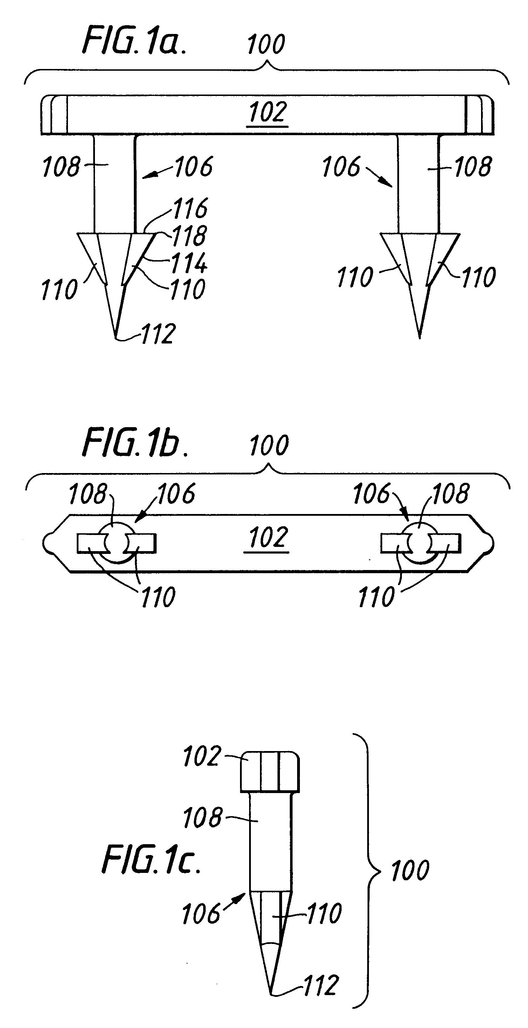

Figs. 1a, 1b and 1c illustrate one embodiment of the present surgical fastener invention. Figs. 1a, 1b, 1c show a two-pronged surgical fastener 100 with barbs 110 on each prong 106. Backspan or base 102 preferably is elongated and can have any shape adapted for its use in an instrument to receive and dispense it, such as the shape as shown, or a rectangular shape. Each prong 106 is an integral part of the fastener and projects substantially perpendicularly from the backspan 102 and in substantially the same direction as each other. The prongs 106 are substantially parallel to each other, and they are located in a spaced apart relationship from the ends of the backspan 102.

The skin tacking fastener of the present invention must function under operating conditions which limit the optimal shape of the prongs. For example the body tissue will be approached from only one direction, i.e., the target body tissue will not be clamped between an anvil and fastener holder. This means the tissue surrounding the fastener implant site will offer less lateral support to prevent bending or twisting. Also, the fastener of the present invention does not have a corresponding retainer portion which, in addition to holding the fastener, also helps to guide and align the fastener prongs as they enter the retainer openings.

Although the fastener of the present invention operates under conditions wherein it has less lateral support while entering the body tissue, one cannot compensate for this difficulty by fabricating it from an inherently strong material like stainless steel: the fastener must be bioabsorbable. Hence, one is presently limited to the strength of the bioabsorbable resins now known.

Prior art bioabsorbable resins such as those designed to lock into corresponding retainer pieces, are not optimally suitable for skin grafting since, because of the shape of their prongs, they encounter high resistance from the body tissue when entering.

It is important, then, for the shape of the prongs to maximize the strength of the prong for a given size fastener, yet minimize the body tissue resistance, while having a means to retain the fastener in the body tissue. Such attributes are achieved herein.

Each prong 106 is composed of a shaft portion 108 and at least one relatively thin substantially triangular shaped barb 110 which is flat on both sides to minimize body tissue resistance. Unlike those fasteners previously used in the art, the blade-like barbs 110 of the present fastener 100 not only puncture body tissue, but also slice through the tissue, thereby easing the entry of the fastener. The barbs 110 are in coplanar alignment with respect to each other and, where a pair of barbs 110 are mounted on a shaft portion 108, they are mounted on opposite sides of each shaft in parallel alignment with the lengthwise axis of the backspan 102. The tip 112 of each shaft 108 is preferably sharply pointed for piercing and entering skin or other body tissue.

The shafts 108 optimally are rounded near the backspan 102, and tapered into the tips 112. The shafts must be at least long enough to penetrate the layer of tissue to be joined and to allow the barbs 110 to become fully embedded in the underlying tissue. They are illustrated in this embodiment as being of equal length, but it is also within the scope of this invention to have unequally sized shafts and barbs. The base 116 of barbs 110 provides a means of retaining the fastener 100 in the body tissue by offering a flat surface highly resistant to movement of the barbs 110 in the direction rearward to that of the insertion of the barbs 110.

Vertices or corner edges 118 of the triangular barbs 110 are formed by the intersection of the base 116 and sides 114. Sides 114 facilitate the insertion of the barbs with minimal skin resistance, whereas vertices 118 further aid in retaining the barbs in the body tissue by digging into the tissue and offering high resistance to rearward movement of the barb.

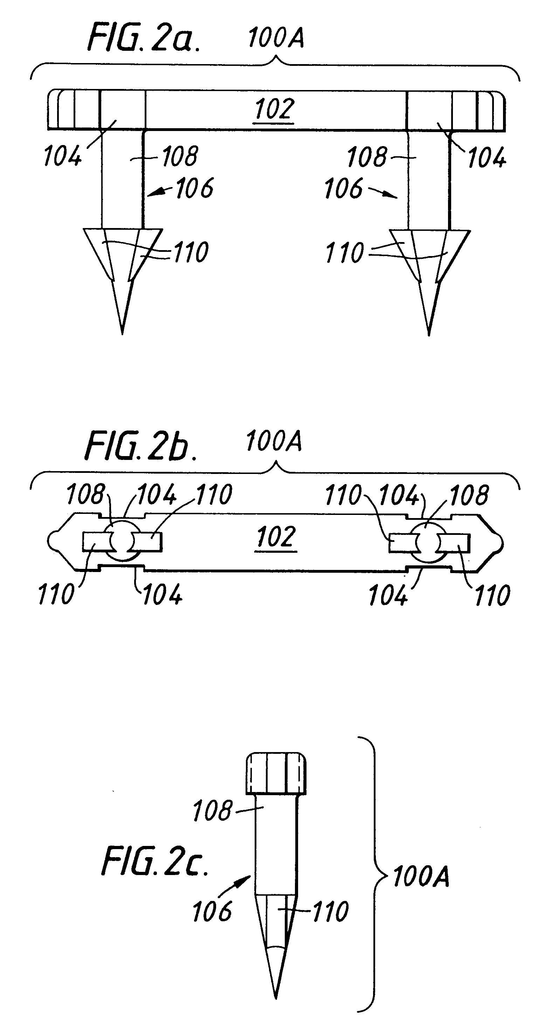

Figs. 2a, 2b, 2c illustrate an alternative embodiment of the present surgical fastener invention 100A including optional indentations 104 in the backspan 102 on both sides of the prongs 106. These indentations 104 can cooperate with rail members in a fastener implanting instrument so as to minimize the possibility of the prongs' bending or twisting as they enter body tissue.

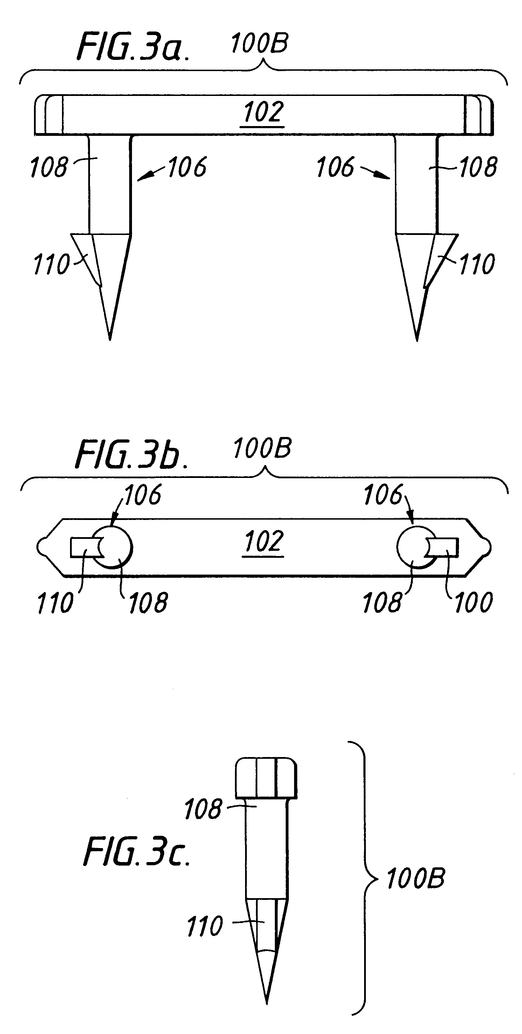

Figs. 3a, 3b, 3c illustrate an alternative embodiment 100B of the present surgical fastener invention including a single barb 110 on each prong, the barb 110 projecting from only one side of the shaft 108.

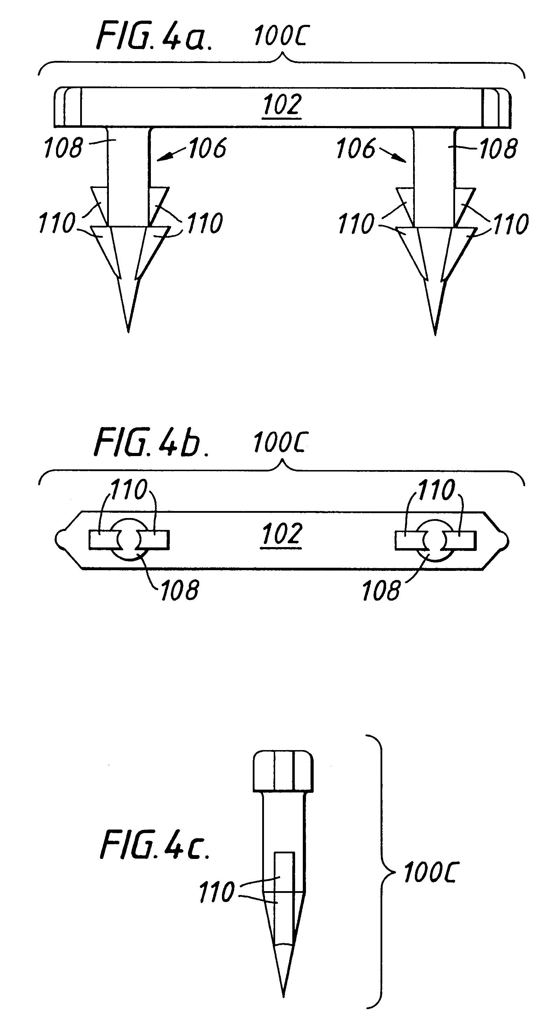

Figs. 4a, 4b, 4c illustrate an alternative embodiment 110C of the present surgical fastener invention including multiple rows of barbs 110 on each prong 106.

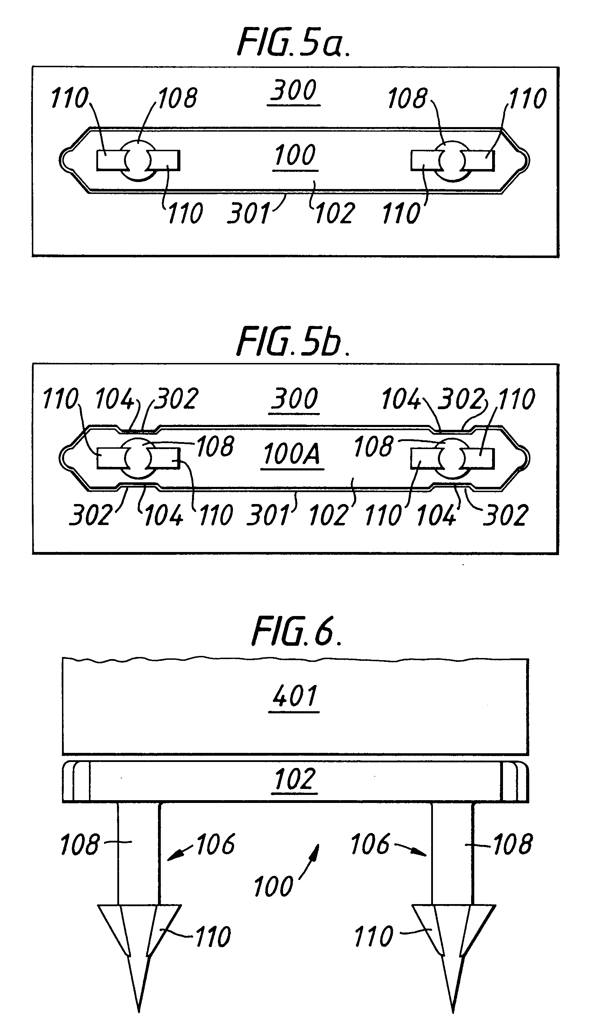

Figs. 5a and 5b illustrate an end view of fasteners 100 and 100A inside an ejection chamber 300 of a surgical stapling apparatus. Fasteners 100 and 100A have prongs 106 pointing towards the exit opening 301. Rail members 302 in Fig. 5b cooperate with indentations 104 of fastener 100A to keep fastener 100A aligned as it is pushed through chamber 300, thereby reducing the possibility of fastener 100A jamming inside the instrument. Also, rails 302 project towards both of the flat sides of barbs 110 thereby deterring them from bending or twisting when they contact the body tissue to be fastened.

It should be realized that indentations 104, and cooperating rail members 302, are optional features which aid in implanting the skin fastener 100, but are not mandatory with respect to the fastener's function of piercing and holding body tissue.

Fig. 6 illustrates a side view showing the fastener 100 in conjunction with a pusher member 401 of a surgical stapling instrument (entire instrument not shown). Such instruments generally have a means to hold one or more fasteners, a trigger means, a means for storing and releasing power such as a spring means, and a drive means to drive the surgical fastener into the tissue. The drive means generally includes a pusher member, such as illustrated in Fig. 6 to push the fastener through an ejection chamber into the body tissue to be fastened.

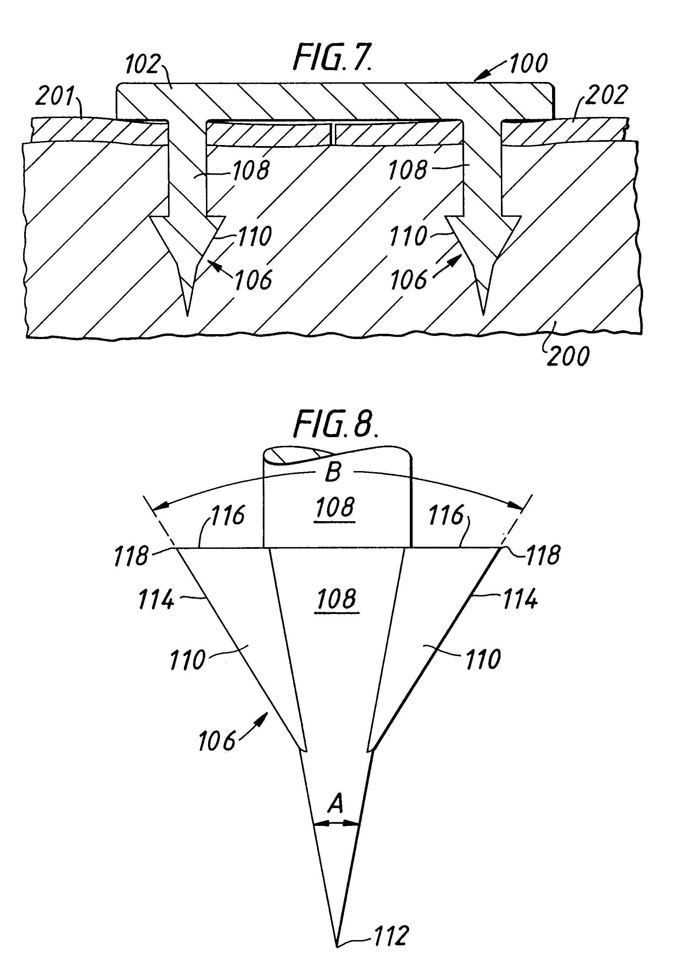

Fig. 7 illustrates the fastener in use. Fastener 100 is embedded in layers of body tissue for grafting skin. Grafted skin 201 is penetrated by a barb and fastened onto base tissue 200. Surrounding skin 202 is penetrated by a second barb. This illustration shows how tissue may be joined in two ways by the present invention: laterally, by bridging the edges of grafted tissue 201 to surrounding tissue 202, and laminarly by pinning a grafted layer 201 onto an underlying base layer of tissue 200. The barbs 110 will not allow the fastener to fall out or be withdrawn.

The fastener of the present invention may have any dimensions suitable for the purpose of fastening body tissue. For example, in one embodiment of the fastener of the present invention it has been found useful to provide a backspan 102 with a length of about 10 mm (0.39 inches), a width of about 1.15 mm (0.045 inches), and a depth of about 0.86 mm (0.034 inches), although other dimensions may also be used. As illustrated in Fig. 7, the length of shaft 108 is typically greater than the depth of grafted tissue 201 and surrounding tissue 202, in order to penetrate the underlying tissue 200 sufficiently, thereby allowing barbs 110 to obtain a firm setting. Thus, in the embodiment being discussed, shaft 108 may be about 4.24 mm (0.167 inches) from the backspan to the tip. The distance between the center points of the shafts 108 are generally about 6.34 mm (0.25 inches), and the distance between the center point of the shaft 108 and the end of the backspan 102 is about 1.78 mm (0.07 inches). As shown in Fig. 8, the angle A of the tip 112 of shaft 108 is preferably about 21°. The angle B formed by the edges of the barbs 110 is preferably about 64°. Side 114 of barb 110 intersects with the tapered portion of shaft 108 at a point about 1.04 mm (0.041 inches) from the tip 112 of shaft 108. Base 116 of barb 110 preferably extends about 0.46 mm (0.018 inches) from shaft 108 to vertex 118. The distance between the base 116 and the point at which side 114 intersects with shaft 108 is about 1.07 mm (0.042 inches), and the distance between the backspan 102 and the base 116 is about 2.13 mm (0.084 inches). As stated above however, other dimensions are obviously suitable in the practice of the present invention.

Because the fastener 100 cannot be easily removed, it is made of a bioabsorbable material such as copolymers of lactide and glycolide, or other bioabsorbable polymer materials. A preferred bioabsorbable resinous material for constructing the surgical fastener of the present invention is disclosed in Kaplan et al., U.S. Patent No. 4,523,591. Fasteners of the present invention may be formed by injection molding of the bioabosrbable materials.

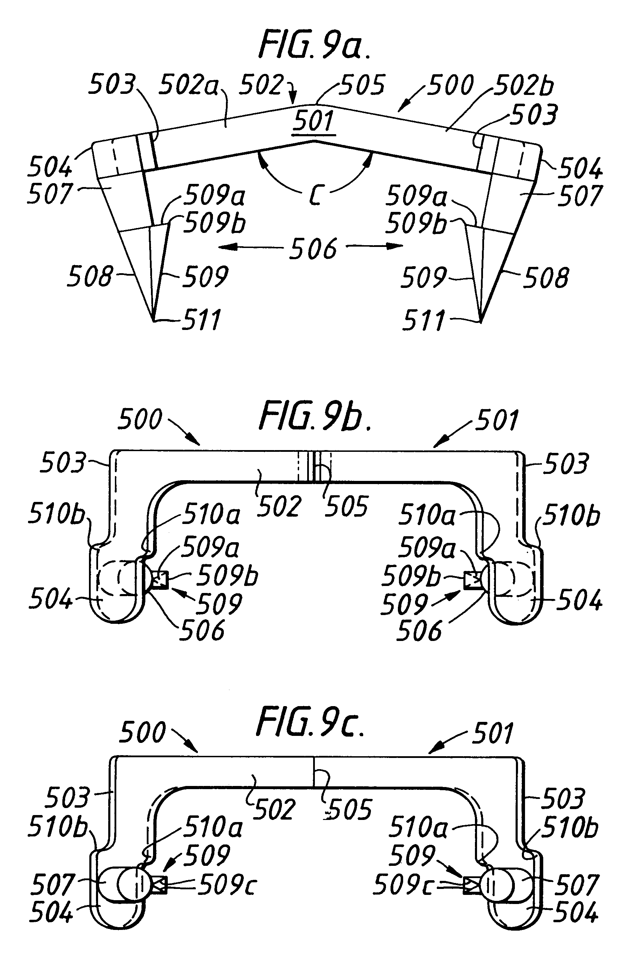

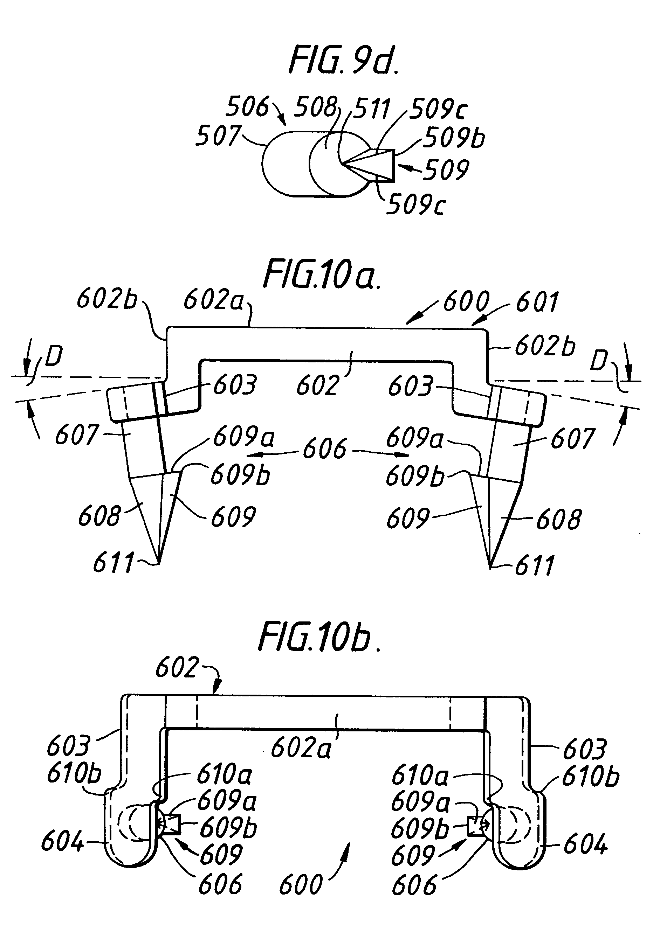

One of the characterising features of the invention is that the distal edge of the barb on the tapered tip of the prong is spaced apart from the distal point of the prong. Figs. 9a, 9b, 9c and 9d show fasteners which lack this particular characterising feature of the present invention. Fastener 500 comprises a backspan 501 and barbed prongs 506. Backspan 501 comprises a bridge portion 502, and preferably at least two arm members 503 extending substantially parallel to each other and transversely in a horizontal direction from bridge 502, each arm extending from the same side of the bridge portion. The arms 503 each comprise an end portion 504 which are in the same plane as the arms, but the end portions 504 are offset in a direction away from each other so that the distance between the end portions 504 is slightly greater than the distance between the arms 503. The arms 503 are of the same width as the end portion 504. Thus, offsetting the end portions produces an inner ridge 510a and an outer ridge 510b. Arms 503 are optional preferred features which provide an offset to facilitate ejection of the fastener from a fastener applying instrument. Armless fasteners are discussed below.

As explained above, in operations such as skin grafting wherein layers of tissue are fastened edge to edge or in closing wounds and incisions, it is desirable to have a lateral force to keep the edges of tissue in close contact. This is accomplished in the present invention by providing a fastener with inwardly biased prongs. In the present embodiment such biasing is accomplished by the bend or flexure 505 midway in the bridge portion 502 of the backspan 501. Bend 505 defines two symmetrical anticlinal wing portions 502a and 502b. As can be seen in Fig. 9a, the bridge 502 is bent at an angel C less than 180°, i.e., the wing portions 502a, 502b each decline between 5° to 25° and optimally 8°22′ from the horizontal. Prongs 506 depend from end portions 504 at right angles to the end portions 504 and arms 503. But since the bridge 502 is slightly bent at flexure 505 the prongs 506 are not parallel to each other. Rather, the prongs are biased inward such that the distal ends of the prongs are closer to each other than the proximal end. As will be explained more fully below, the fastener functions like a spring clamp to hold the edges of the tissue together.

Each prong 506 comprises a shaft 507, and tip 508 which optimally tapers into a sharp point 511. Shaft 507 is slightly tapered from an oval cross section near the backspan 501, to a circular cross section at the tip 508.

Each prong 506 has at least one substantially triangular shaped barb 509 which is flat on both sides to minimize body tissue resistance. Each barb 509 is mounted on the interior side of the prong 506 such that they face each other. The blade-like barbs 509 of the present fastener 500 not only puncture body tissue, but also slice through the tissue, thereby easing the entry of the fastener. The barbs 509 are in coplanar alignment with respect to each other. Barbs 509 each have a flat back surface 509a and vertex 509b to provide resistance to backward movement of the fastener 500. Once implanted, fastener 500 cannot be easily removed. Barbs 109 also have cutting edges 509c to facilitate implantation by slicing through body tissue.

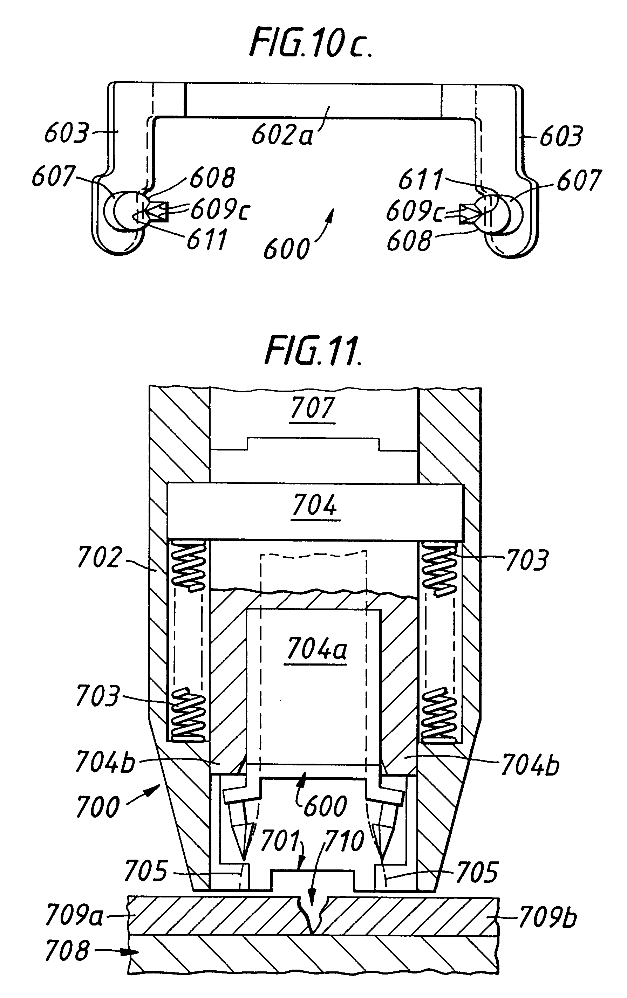

Referring now to Figs. 10a, 10b and 10C which show respectively the front side view, top and bottom views of an alternative embodiment 600 of the present invention, backspan 601 differs from the previously described embodiment in that backspan 601 has a U-shaped bridge 602 with a top beam 602a, and downwardly extending sidewalls 602b. Top beam 602a does not have a biasing bend or flexure similar to flexure 505 in bridge 502 of the previously described embodiment. Instead, the top beam 602a is straight. Arms 603 of backspan 601 are tilted from horizontal, however, at an angle D which is optimally 5° to 25° such that the prongs 606 are biased inward. The side walls 602b allow an expansion space under the top beam 602a in order to allow for swelling of the body tissue.

As in the previously described embodiment, arms 603 have end portions 604 which are offset a small distance outwardly, and away from each other, thereby creating inner ridges 610a and outer ridges 610b. Also, arms 603 are preferable, optional, features of the fastener 600.

Prongs 606 depend from end portions 604 at substantially right angles to the end portions 604. Each prong 606 comprises a shaft 607 and tip 608 which optimally tapers to a sharp point 611. Furthermore, as in the previously described embodiment, each prong 606 has at least one substantially triangularly shaped barb 609 which is flat on both sides. Each barb 609 is mounted on the interior side of the prong 606. The blade like barbs 609 not only puncture body tissue, but also slice through the tissue, thereby facilitating the entry of the fastener. The barbs 609 are in coplanar alignment with respect to each other.

Barbs 609 each have a flat back surface 609a and vertex 609b to provide resistance to the backward movement of the fastener 600. Once implanted, fastener 600 cannot be easily removed. Barbs 609 also have cutting edges 609c to facilitate implantation of the fastener 600 by slicing through body tissue.

Because the fasteners disclosed herein are not adapted to be removable they are preferably made of bioabsorbable material such as copolymers of lactide and glycolide, or other bioabsorbable polymer materials. A preferred bioabsorbable resinous material for constructing fasteners 500 and 600 is disclosed in Kaplan et al. U.S. Patent No. 4,523,591.
Fasteners of the present invention may be formed as integral, single piece constructions by injection molding. The material of construction for fasteners 500 and 600 must have a degree of resiliency or springiness sufficient to allow bending or flexing of the fasteners 500 or 600 from the initial angled position to a straight position when they are implanted. When being driven by a fastener implanting instrument the backspan 501 or 601 will straighten such that the prongs 506 or 606 will enter the body tissue perpendicularly. When the driving force is removed the resiliency of the construction material will urge the fastener back into the initially bent position thereby biasing the prongs 506 or 606 inwardly. When implanted across a wound or an incision the fasteners will exert a lateral inward force to clamp the two edges of the tissue together.

Fig. 11 illustrates a fastener 600 in combination with a pusher mechanism of a fastener applying instrument 700. The instrument walls 702 enclose a pusher plate 704 having a backspan pusher member 704a and a pair of arm pusher members 704b. Pusher plate 704 is adapted to contact the top surface of the backspan and arms as the fastener 600 is being implanted. Driver 707 is adapted to drive the pusher plate 704. Resilient springs 703 return the pusher plate 704 to the initial pre-firing position after the fastener 600 has been ejected. Camming surfaces 705 contact the barbs and bias the prongs into a substantially parallel orientation with respect to each other and a substantially perpendicular orientation with respect to the body tissue 708. The offset provided by the arms 503, and 603 permit the prongs to be cammed into a vertical position and parallel orientation without interfering with the downward movement of the fastener. Exit opening 701 allows egress of the fastener 600. Fastener 600 (or alternatively fastener 500) can be used to fasten body tissue laterally as, for example, in closing wound 710 between tissue layers 709a and 709b, and it can also be used to fasten body tissue laminarly, as for example in grafting body tissue layers 709a and 709b onto body tissue layer 708.

The prongs of the above described fasteners, are oriented obliquely to each other such that the distal ends of the prongs are inclined towards each other. The oblique orientation of the prongs can be provided by flexure 505 or by a tilt in the arm members 603, as mentioned above. The fastener has sufficient flexibility to enable the prongs to move to a substantially parallel orientation to each other when pressure is applied, for example by camming surface 705. Straightening the prongs to a parallel orientation permits the fastener to enter the body tissue more easily. When the fastener has been implanted and is no longer contacted by the camming surface, the resiliency of the fastener urges the prongs back into their initial oblique orientation, thereby acting as a spring clamp to close up wounds in tissue and hold the edges of torn body tissue in close adjacency for quicker and easier healing. Barbs are located on the inside surface of the prongs to grip the body tissue more securely.

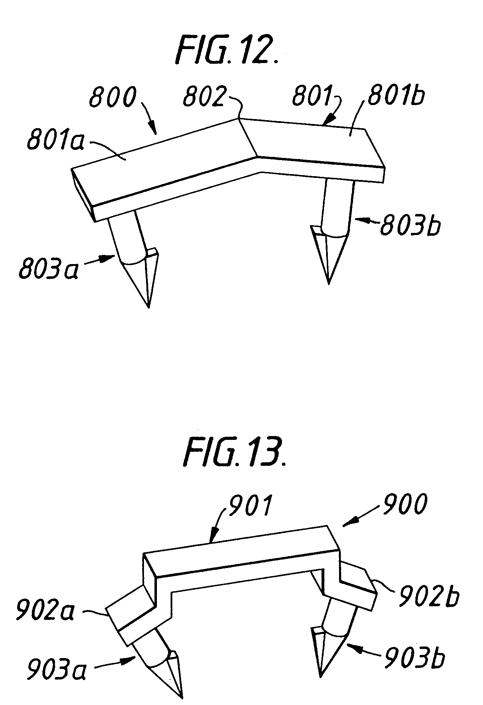

As can be seen in Figs. 12 and 13 the inwardly biased skin fastener can be constructed without arms 503 or 603, although the arms are preferable because they provide an offset to facilitate camming the prongs to a vertical position in the fastener applying instrument. Armless fastener 800 defines a single vertical plane, and is an armless alternative embodiment to fastener 500. Backspan 801 is divided into anticlinal portions 801a and 801b, by a central bend 802. Prongs 803a and 803b depend perpendicularly from their respective portions of the backspan, and their distal ends are biased towards each other by means of the bend 802 in the backspan. Armless fastener 900 also defines a single plane, but it has a U-shaped backspan 901, inclined end portions 902a and 902b, and prongs 903a and 903b, which are inclined so that their respective distal ends are biased towards each other.

The surgical fasteners of the present invention can be made of any size suitable to the purpose of fastening body tissue. Generally, the backspan may be about 7 to 10mm in length, for example, and the prongs may be about 3 to 5mm long. The arms may be about 2 to 4mm from the backspan to the prong. Other dimensions may be used in accordance with the different types of applications intended.

## Claims

1. A surgical fastener (100) of bioabsorbable material for joining layers of body tissue comprising:
a) a backspan (102); and
b) at least two prongs (106) extending substantially perpendicularly from said backspan, each prong having a shaft portion (108) and at least one substantially triangular shaped barb (110) for penetrating body tissue and retaining the fastener in the body tissue whereby said barb when in the body tissue retains the fastener in the body tissue without engaging a retainer;
and characterised in that:
i) the barb (110) is blade-like and thinner than its respective prong (106);
ii) the prongs (106) are spaced apart from the ends of the backspan (102);
iii) the shaft portion (108) has a tip portion tapering into a distal point (112) and said barb (110) is located on the tapered tip portion of said shaft portion (108) and has a distal edge spaced apart from the distal point (112); and
(iv) the prongs (106) are either in substantially parallel relationship or in oblique orientation to each other.

2. A surgical fastener as claimed in claim 1, wherein each prong (106) has at least two barbs (110) which are flat on both sides and in coplanar alignment in relation to each other.

3. A surgical fastener as claimed in claim 1 or 2 wherein
a) the backspan comprises a bridge member (502); and
b) the at least two prongs (506) depend from the backspan, said prongs being in oblique orientation to each other in which the distal ends (511) of said prongs are inclined toward each other and spaced apart, said prongs being movable in response to pressure to a substantially parallel orientation to each other, and being resiliently returnable to said oblique spaced apart orientation when said pressure is removed.

4. A surgical fastener as claimed in claim 3 wherein said backspan additionally comprises at least two arms (503) extending substantially horizontally from said backspan in substantially parallel orientation to each other.

5. A surgical fastener as claimed in claim 4, wherein the bridge member (502) is an inverted U-shaped structure, and said arms (503) are inclined with respect to said backspan.

6. A surgical fastener as claimed in any one of claims 3 to 5 wherein the bridge member (503) comprises a central bend (505) defining two anticlinal portions.

7. A surgical fastener as claimed in any one of the preceding claims, wherein each prong (106) has at least one barb (110) on its shaft portion (108) in addition to the said at least one barb (110) on the tip portion of the prong.

8. A surgical fastener as claimed in any one of the preceding claims, having said prongs extending in the same direction as each other, in combination with a surgical stapler having means for implanting a surgical fastener into body tissue.

9. A surgical fastener as claimed in claim 8, said surgical stapler further including a chamber for holding at least one surgical fastener (500), means for storing and releasing power, and trigger means, the surgical fastener (500) comprising:
a) a backspan (501) comprising a bridge member (502); and
b) at least two prongs (506) depending from the backspan, said prongs being in oblique orientation to each other in which the distal ends of said prongs (511) are inclined towards each other, said prongs being movable in response to pressure to a substantially parallel orientation to each other, and being resiliently returnable to said oblique orientation when said pressure is removed.

10. A surgical fastener as claimed in claim 9 including means to apply said pressure to the fastener to move the prongs to said substantially parallel orientation during entry into bodily tissue.

## Patentansprüche

1. Chirurgisches Befestigungselement (100) aus biologisch absorbierbarem Material zum Verbinden von Körpergewebeschichten mit:
a) einem Rückensteg (102); und
b) mindestens zwei Zinken (106), die sich im wesentlichen senkrecht von dem Rückensteg aus erstrecken, wobei jede Zinke einen Schaftabschnitt (108) und mindestens einen im wesentlichen dreieckig geformten Widerhaken (110) zum Durchdringen des Körpergewebes und zum Zurückhalten des Befestigungselementes in dem Körpergewebe enthält, wodurch der Widerhaken, wenn er sich in dem Körpergewebe befindet, das Befestigungselement in dem Körpergewebe ohne Eingriff in ein Rückhalteelement zurückhält; und
dadurch gekennzeichnet, daß
i) der Widerhaken (110) klingenförnig und dünner als sein entsprechender Zinken (106) ist;
ii) die Zinken (106) von den Enden des Rückenstegs (102) beabstandet sind;
iii) der Schaftabschnitt (108) einen Spitzenabschnitt aufweist, der sich in einen distalen Punkt (112) verjüngt, und der Widerhaken (110) auf dem verjüngten Spitzenabschnitt des Schaftabschnittes (108) angeordnet ist und eine von dem distalen Punkt (112) beabstandete distale Kante aufweist; und
iv) die Zinken (106) entweder im wesentlichen in paralleler Beziehung oder in schräger Ausrichtung zueinander stehen;

2. Chirurgisches Befestigungselement nach Anspruch 1, wobei jeder Zinken (106) mindestens zwei Widerhaken (110) besitzt, welche an beiden Seiten flach sind und in komplanarer Beziehung zueinander stehen.

3. Chirurgisches Befestigungselement nach Anspruch 1 oder 2, wobei:
a) der Rückensteg ein Brückenelement (502) aufweist; und
b) die mindestens zwei Zinken (506) sich von dem Rückensteg weg erstrecken, wobei die Zinken in schräger Ausrichtung zueinander stehen, in welcher die distalen Enden (511) der Zinken zueinander geneigt und beabstandet sind, die Zinken als Reaktion auf Druck in eine im wesentlichen parallele Ausrichtung zueinander bewegt werden können, und elastisch in die schräge beabstandete Ausrichtung zurückkehren können, wenn der Druck weggenommen wird.

4. Chirurgisches Befestigungselement nach Anspruch 3, wobei der Rückensteg zusätzlich mindestens zwei Arme (503) aufweist, die sich im wesentlichen horizontal von dem Brückensteg weg in im wesentlichen paralleler Ausrichtung zueinander erstrecken.

5. Chirurgisches Befestigungselement nach Anspruch 4, wobei das Brückenelement (502) eine umgedrehte U-förmige Struktur ist, und die Arme (503) bezogen auf den Brückensteg geneigt sind.

6. Chirurgisches Befestigungselement nach einem der Ansprüche 3 bis 5, wobei das Brückenelement (503) eine zentrale Biegestelle (505) enthält, welche zwei gegeneinander geneigte Abschnitte definiert.

7. Chirurgisches Befestigungselement nach einem der vorstehenden Ansprüche, wobei jeder Zinken (106) mindestens einen Widerhaken (110) an seinem Schaftabschnitt (108) zusätzlich zu dem mindestens einen Widerhaken (110) an den Spitzenabschnitt des Zinken besitzt.

8. Chirurgisches Befestigungselement nach einem der vorstehenden Ansprüche, mit den sich in der derselben Richtung aufeinander zu erstreckenden Zinken in Verbindung mit einem chirurgischen Klammerungsgerät mit einer Einrichtung zum Implantieren eines chirurgischen Befestigungselementes in Körpergewebe.

9. Chirurgisches Befestigungselement nach Anspruch 8, wobei das chirurgische Klammerungsgerät ferner eine Kammer zum Halten mindestens eines chirurgischen Befestigungselementes (500), eine Einrichtung zum Speichern und Freigeben von Energie und eine Auslöseeinrichtung enthält, und das chirurgische Befestigungselement (500) aufweist:
a) einen Rückensteg mit einem Brückenelement (502); und
b) mindestens zwei Zinken (506), die sich von dem Rückensteg weg erstrecken, wobei die Zinken in schräger Ausrichtung zueinander stehen, in welcher die distalen Enden (511) der Zinken zueinander geneigt sind, die Zinken als Reaktion auf Druck in eine im wesentlichen parallele Ausrichtung zueinander bewegt werden können, und elastisch in die schräg beabstandete Ausrichtung zurückkehren können, wenn der Druck weggenommen wird.

10. Chirurgisches Befestigungselement nach Anspruch 9 mit einer Einrichtung zum Anwenden des Drucks auf das Befestigungselement, um die Zinken in die im wesentlichen parallele Ausrichtung während des Eindringens in Körpergewebe zu bringen.

## Revendications

1. Attache chirurgicale (100) en un matériau bioabsorbable pour relier les couches d'un tissu corporel comprenant :
a) une portée arrière (102); et
b) au moins deux pointes (106) s'étendant sensiblement perpendiculairement de ladite portée arrière, chaque pointe possédant une partie d'arbre (108) et au moins une barbe sensiblement triangulaire (110) pour pénétrer dans le tissu corporel et pour retenir l'attache dans le tissu corporel par quoi ladite barbe, lorsqu'elle se trouve dans le tissu corporel, retient l'attache dans le tissu corporel sans la mise en prise avec un élément de retenue;
et caractérisée en ce que :
i) la barbe (110) est en forme de lame et plus mince que sa pointe respective (106);
ii) les pointes (106) sont espacées des extrémités de la portée arrière (102);
iii) la partie d'arbre (108) présente une partie d'extrémité diminuant coniquement en une pointe distale (112) et ladite barbe (110) est localisée sur la partie d'extrémité conique de ladite partie d'arbre (108) et présente un bord distal espacé de la pointe distale (112); et
iv) les pointes (106) sont soit dans une relation sensiblement parallèle soit dans une orientation oblique l'une à l'autre.

2. Attache chirurgicale selon la revendication 1, dans laquelle chaque pointe (106) possède au moins deux barbes (110) qui sont plates des deux côtés et en un alignement coplanaire l'une relativement à l'autre.

3. Attache chirurgicale selon la revendication 1 ou 2, dans laquelle
a) la portée arrière comprend un élément formant pont (502); et
b) les au moins deux pointes (506) s'étendent vers le bas depuis la portée arrière, lesdites pointes étant dans une orientation oblique l'une à l'autre dans laquelle les extrémités distales (511) desdites pointes sont inclinées l'une vers l'autre et espacées, lesdites pointes étant déplaçables en réponse à une pression à une orientation sensiblement parallèle l'une à l'autre et pouvant être ramenées élastiquement à ladite orientation oblique espacée lorsque ladite pression est retirée.

4. Attache chirurgicale selon la revendication 3, dans laquelle ladite portée arrière comprend additionnellement au moins deux bras (503) s'étendant sensiblement horizontalement depuis la portée arrière suivant une orientation sensiblement parallèle l'un à l'autre.

5. Attache chirurgicale selon la revendication 4, dans laquelle l'élément formant pont (502) est une structure en forme de U inversé, et lesdits bras (503) sont inclinés relativement à ladite portée arrière.

6. Attache chirurgicale selon l'une des revendications 3 à 5, dans laquelle l'élément formant pont (503) comprend une courbure centrale (505) définissant deux parties anticlinales.

7. Attache chirurgicale selon l'une des revendications précédentes, dans laquelle chaque pointe (106) possède au moins une barbe (110) sur sa partie d'arbre (108) en plus de ladite au moins une barbe (110) sur la partie d'extrémité de la pointe.

8. Attache chirurgicale selon l'une des revendications précédentes, dans laquelle lesdites pointes s'étendent dans la même direction l'une comme l'autre, en combinaison avec une agrafeuse chirurgicale pourvue d'un moyen pour implanter une attache chirurgicale dans le tissu corporel.

9. Attache chirurgicale selon la revendication 8, ladite agrafeuse chirurgicale comprenant en outre une enceinte pour retenir au moins une attache chirurgicale (500), un moyen pour stocker et libérer l'énergie et un moyen de déclenchement, l'attache chirurgicale (500) comprenant :
a) une portée arrière (501) comprenant un élément formant pont (502); et
b) au moins deux pointes (506) s'étendant vers le bas depuis la portée arrière, lesdites pointes étant dans une orientation oblique l'une à l'autre dans laquelle les extrémités distales desdites pointes (511) sont inclinées l'une vers l'autre, lesdites pointes étant déplaçables en réponse à une pression suivant une orientation sensiblement parallèle l'une à l'autre et pouvant être ramenées élastiquement à ladite orientation oblique lorsque ladite pression est retirée.

10. Attache chirurgicale selon la revendication 9, incluant un moyen pour appliquer ladite pression à l'attache pour déplacer les pointes suivant ladite orientation sensiblement parallèle pendant l'entrée dans le tissu corporel.
